# EUROPEAN PATENT APPLICATION

(11) **EP 1 939 283 A1**
(43) Date of publication of application: **02.07.2008**
(21) Application number: 06810820.8
(22) Date of filing: 29.09.2006
(51) Int. Cl.: C12N 9/74, C12N 5/10, C12N 15/09

(54) **PROCESS FOR PRODUCING RECOMBINANT HUMAN THROMBIN BY CULTURED CELL**

(30) Priority: 30.09.2005 JP 2005289076
(71) Applicant: Juridical Foundation The Chemo-Sero-Therapeutic Research Institute, Kumamoto-shi Kumamoto-ken 860-8568 (JP)
(72) Inventor: ONCHI, Tatsufumi, c/o JURIDICAL FOUNDATION THE CHEMO-SERO-THERAPEUTI C RESEARCH INSTITUTE, KIKUCHI RESEARCH CENTER,, Kikuchi-shi, Kumamoto-ken 869-1298 (JP); NAKAHARA, Yo, c/o JURIDICAL FOUNDATION THE CHEMO-SERO-THERAPEUTI C RESEARCH INSTITUTE, KIKUCHI RESEARCH CENTER,, Kikuchi-shi, Kumamoto-ken 869-1298 (JP); IMAMURA, Takayuki, Kikuchi-shi, Kumamoto-ken 869-1298 (JP); NOUCHI, Toshinobu, Kikuchi-shi, Kumamoto-ken 869-1298 (JP); NAKATAKE, Hiroshi, Kikuchi-shi, Kumamoto-ken 869-1298 (JP)
(74) Representative: Vossius & Partner
(86) International application number: PCT/JP2006/319414
(87) International publication number: WO 2007/040162

(57) **Abstract**

The present invention provides for a process for preparing a recombinant human thrombin. A process for preparing a recombinant human thrombin which comprises: (1) obtaining a transfectant cell producing a human prothrombin by introducing an expression vector, wherein a gene fragment coding for a human prothrombin gene is incorporated, into an animal cell; (2) purifying a human prothrombin from the culture of the transfectant cell above by an anion exchanger; (3) converting the purified human prothrombin into a human thrombin by subjecting said human prothrombin to the action of ecarin; and (4) purifying the human thrombin from the solution after treatment with ecarin by an affinity method using benzamidine and a cation exchanger, and human thrombin obtained by said process, and a CHO cell that produces human prothrombin.

## Description

### TECHNICAL FIELD

The present invention relates to a human thrombin obtained by a genetic recombination technique and a process for preparing the same. More specifically, the present invention relates to a process for preparing a recombinant human thrombin which comprises obtaining CHO cells producing a human prothrombin, purifying a human prothrombin from the culture of said cells by an anion exchange chromatography, treating the obtained human prothrombin with ecarin to convert it into a human thrombin, and purifying said human thrombin by an affinity chromatography employing benzamidine and a cation exchanger, and a human thrombin obtained by said process. The human thrombin of the present invention belongs to the field of a medicament for clinical usage and is used as a hemostatic. The present invention also relates to human prothrombin producing cells obtained in the process for preparing a human thrombin as well as a process for preparing a human thrombin and a human thrombin obtained by said process.

### BACKGROUND ART

Thrombin is a trypsin-like serine protease having the activity essential to maintenance and development of life such as formation of hemostatic thrombus or curing of wound. Thrombin includes meizothrombin, α-thrombin, β-thrombin and γ-thrombin, among which α-thrombin is most important from the physiological point of view. Prothrombin, a precursor of thrombin, is biosynthetically produced in the hepatocytes in a vitamin K dependent manner and its blood level is in a range of 100 to 150 µg/ml. A vitamin K dependent coagulation factor has a Gla containing region (Gla domain) at the N-terminal and binds to a phospholipid via Ca²⁺ ion. It is known that, upon binding of the Gla domain to Ca²⁺ ion, a high dimensional overall structure of the protein is altered to thereby exert a key function as interacting with cofactors.

Prothrombin undergoes activation by FXa-FVa complex on the phospholipids of the cellular membrane wherein Arg320-Ile321 bonding in prothrombin is cleaved through restricted cleavage to form meizothrombin having the Gal domain and Kringle domain. Subsequently, Arg271-Thr272 bonding is cleaved through restricted cleavage to form α-thrombin, which is released from the cellular membrane and exhibits a variety of physiological activities by restrictedly cleaving a number of plasma proteins or various thrombin receptors on the cellular membrane. α-Thrombin is a two-chained molecule consisting of A chain and B chain. When B chain essential to the enzymatic activity undergoes autolysis, β-thrombin or γ-thrombin is produced to thereby lose an ability to activate fibrinogen or platelets.

Most of the formed thrombin participates locally in formation of larger thrombus by binding to fibrin clot. α-Thrombin not only converts fibrinogen into fibrin but also activates FXIII to trigger cross-linkage of fibrin. Besides, α-thrombin may accelerate coagulation by activating FVIII and FV, a cofactor of FIX and FXa, respectively, to proceed coagulation. Thus, thrombin plays an important role in hemostasis and hence is a highly useful protein for use as a hemostatic.

On the other hand, thrombin changes its substrate specificity upon binding to thrombomodulin on the vascular endothelial cells to activate Protein C to promote anticoagulation. Thus, utilizing this enzymatic activity, thrombin is also useful as a process enzyme for preparing activated Protein C, an anticoagulant. Moreover, α-thrombin may potently coagulate and activate platelets and also exhibits a mitogenic activity. α-Thrombin, as displaying such a variety of physiological activities, has been used as a reagent in various fields of research and expected to still increase its utility in future.

With such functions and activities, thrombin has been widely used as a hemostatic, a process enzyme or a reagent for research. For example, for hemorrhage at the upper gastrointestinal tracts, thrombin derived from blood has been endoscopically spread or orally administered with successful hemostasis. Also, a fibrin glue used as a tissue adhesive comprises thrombin derived from blood together with fibrinogen and FXIII.

However, the thrombin materials described above are isolated from human or bovine blood and hence may also contain various dangerous factors derived from source blood that are considered to exert adverse effects on human. There is a fear that e.g. virus causing hepatitis such as HAV, HBV, HCV, HEV or TTV, virus causing immunodeficiency diseases such as HIV, or abnormal prion causing CJD etc. are present in the thrombin materials. In fact, drug-induced disaster caused by blood products contaminated with these dangerous factors has been a big social problem. Moreover, since human or bovine blood is derived from living material, there is no guarantee that it is stably provided. This is, in view of drugs, particularly an important and severe problem that must urgently be solved.

According to the conventional method, thrombin is prepared by activating a precursor of thrombin with FXa derived from blood, i.e. blood-derived FXa is used for the activation process. Thus, even if a precursor of thrombin is prepared by the genetic engineering technique, as far as blood-derived FXa is used as the activating enzyme, a fear of contamination of blood components cannot be excluded. For preparing FXa by the genetic engineering technique, a precursor thereof, FX, must be prepared by the genetic engineering technique and activated with FIXa. Further, for preparing FIXa by the genetic engineering technique, a precursor thereof, IX, must be prepared by the genetic engineering technique and activated with another coagulation factor. In this way, unless the most upstream enzyme in the cascade of coagulation reaction is prepared by the genetic engineering technique, thrombin cannot be prepared that is free from danger of contaminated blood components.

Taking into consideration a risk and limitation due to the use of blood as a source material for preparing thrombin and activating enzymes, alternative source and method allowing for provision of thrombin in a safer and more stable manner is desired. With this background, expression of thrombin has been reported using microorganism or animal cells as a host.

However, the conventional methods are disadvantageous in that, for example, thrombin expressed in E. coli forms aggregation, called inclusion body, which makes it difficult to recover thrombin. Specifically, to dissolve aggregation and to refold therefrom a functional protein is much inefficient and hence is not worthwhile to be applied to industrial usage (cf. e.g. Non-patent reference 1). Besides, expression of various thrombin precursors in culture cells has been reported but with a low expression level, the highest level being 25 µg/mL, and hence is not suitable for industrial application (cf. e.g. Patent reference 1, and Non-patent references 2, 3, 4, 5 and 6).

The present inventors have earnestly continued research activities with respect to a thrombin precursor gene, a signal peptide, an expression plasmid, a host cell and a culture medium and as a result have found, as disclosed in WO2003/004641, a process for high expression of a thrombin precursor using SP2/0 cells and CHO cells as a host. With this process, the present inventors have succeeded in obtaining cells expressing a high level of a thrombin precursor, i.e. recombinant SP2/0 cells which had an expression level of 100 µg/ml in suspension culture using a 250 ml spinner flask with a serum free culture and CHO cells with an expression level of 110 µg/ml in stationary culture using a culture medium with 10% serum. Besides, the present inventors have also found a process for preparing an enzyme ecarin for use in activation of a thrombin precursor to thrombin by a genetic engineering technique. In addition, the present inventors have found a method for purifying thrombin to high purity at a final purification yield of 40% from a 2000 ml culture containing a thrombin precursor. However, from the viewpoint of an industrial scale production, such as production of a recombinant thrombin with hundreds or more litters of culture, in a stable manner at low cost, further development in production technique will be unavoidable. Thus, development has been desired in establishment of cell lines which have high stability through passage and are suitable for the production of a recombinant thrombin, selection of a serum-free, inexpensive culture medium as well as adaptation of cells to such a medium, adjustment of adhesive cells to suspension culture, setup of conditions for various controls in a tank culture in hundreds or more litters, and purification at a higher yield and at a lower cost.

For some proteins with a sugar chain, their biological activity is affected by a kind and an amount of their sugar chain. For instance, a sugar chain is not added to an EPO protein when expressed in E. coli whereas a large amount of mannose-type N-linked sugar chain is added when expressed in yeast cells. In both cases, the protein is reported to exhibit a lowered biological activity (cf. e.g. Non-patent reference 7). After subsequent study, it was found that thrombin obtained by the process disclosed in WO2003/004641 as described above wherein prethrombin is expressed in SP2/0 cells had sugar chains with addition of N-glycolylneuraminic acid. However, A. Noguchi et al. have pointed out that N-glycolylneuraminic acid exhibits antigenicity and immunogenicity to human (cf. e.g. Non-patent reference 8). Thus, for the production of a protein for clinical and medical usage, it will be important to select a host by taking into consideration not only increase in production scale and reduction of production cost but also maintenance of its biological activity and safety when administered to human being.
Patent reference 1: W093/013208
Non-patent reference 1: J. Biol. Chem. 270, 163-169, 1995
Non-patent reference 2: J. Biochem. 262, 6792-6734(1987)
Non-patent reference 3: J. Biochem. 266, 13796-13803(1991)
Non-patent reference 4: J. Biochem. 266, 9598-9604(1991)
Nan-patent reference 5: Proc Natl. Acad. Sci. U.S.A. 88, 6775-6779(1991)
Non-patent reference 6: Protein. Exp. Purif. 10, 214-225(1997)
Non-patent reference 7: Gene 79, 167-180, 1989
Non-patent reference 8: J. Biochem. 117, 59-62(1995)

### DISCLOSURE OF THE INVENTION

### (Technical Problem to be Solved by the Invention)

A human thrombin is a useful protein as a hemostatic in the field of a medicament for clinical usage. As described above, it is desired to develop a process for the production of a human thrombin providing the same in a safe and stable manner at low cost by removing various risk factors possibly present in blood.

The present inventors have established in the previously filed patent application WO2003/004641 a process for the production of a human thrombin providing the same in a safe and stable manner at low cost. After the subsequent study, however, it was found that still further development would be necessary for its production in a large, industrial scale of several hundreds or more litters.

An object of the present invention is to provide an industrial process for preparing a recombinant human thrombin by means of a specific combination of various techniques including establishment of cell lines for its production and improvement in culture and purification process.

### (Means for Solving the Problems)

Under the circumstances, the present inventors have earnestly continued research activities in order to solve the problems described above and as a result found that thrombin obtained from expression in SP2/0 cells had sugar chains with addition of N-glycolylneuraminic acid whereas one obtained from expression in CHO host cells had sugar chains with addition of N-acetylneuraminic acid, the same sialic acid as that found in a human thrombin derived from plasma. Besides, although the CHO cells disclosed in WO2003/004641 above, having adhesive property, could not be subject to suspension culture and thus was not industrially practical, the present inventors have found transfectant CHO cells expressing a human prothrombin capable of suspension culture so as to enable serum free, full suspension culture at a large scale. It was demonstrated that the transfectant had as high an expression level as 128 µg/mL and the thrombin expressed therefrom had sugar chains with addition of N-acetylneuraminic acid, the same sialic acid as that found in a human thrombin derived from plasma. Furthermore, by combining optimal processes for purification, the present inventors have completed an industrial process for preparing a recombinant human thrombin at an industrial culture scale of 500 L or more at a low cost wherein the recombinant human thrombin produced therefrom has higher biocompatibility than that with addition of N-glycolylneuraminic acid.

Thus, the present invention provides a process for preparing a recombinant human thrombin as described hereinbelow.
1. A process for preparing a recombinant human thrombin which comprises a series of the following steps (1) to (4):
   (1) a step of obtaining a transfectant cell producing a human prothrombin by introducing an expression vector, wherein a gene fragment coding for a human prothrombin gene is incorporated, into an animal cell;
   (2) a step of purifying a human prothrombin from the culture of the transfectant cell above by an anion exchanger;
   (3) a step of converting the purified human prothrombin into a human thrombin by subjecting said human prothrombin to the action of ecarin; and
   (4) a step of purifying the human thrombin from the solution after treatment with ecarin by an affinity method using benzamidine and a cation exchanger.
2. The process according to 1 wherein the animal cell is a CHO cell.
3. The process according to 1 or 2 wherein the human prothrombin gene consists of the nucleotide sequence as depicted in SEQ ID NO: 3.
4. A process for preparing a recombinant human prothrombin which comprises a series of the following steps (1) to (2):
   (1) a step of obtaining a transfectant cell producing a human prothrombin by introducing an expression vector, wherein a gene fragment coding for a human prothrombin gene is incorporated, into an animal cell; and
   (2) a step of purifying a human prothrombin from the culture of the transfectant cell above by an anion exchanger.
5. The process according to 4 wherein the animal cell is a CHO cell.

The present invention also provides a human thrombin obtained by the process as set forth in any of 1 to 3 above, a human prothrombin obtained by the process as set forth in any of 4 to 5 above, and the CHO cell producing a human thrombin.

### (More Efficacious Effects than Prior Art)

According to the present invention, a process for preparing a recombinant human thrombin, which has a sugar chain structure of N-acetylneuraminic acid, the same as that of a human thrombin derived from plasma, is provided.

### BEST MODE FOR CARRYING OUT THE INVENTION

The present invention is characterized by a process for preparing a recombinant human thrombin which comprises (1) a step of obtaining a transfectant cell producing a human prothrombin by introducing an expression vector, wherein a gene fragment coding for a human prothrombin gene is incorporated, into an animal cell; (2) a step of purifying a human prothrombin from the culture of the transfectant cell above by an anion exchanger; (3) a step of converting the purified human prothrombin into a human thrombin by subjecting said human prothrombin to the action of ecarin; and (4) a step of purifying the human thrombin from the solution after treatment with ecarin by an affinity method using benzamidine and a cation exchanger, and a process for preparing a recombinant human prothrombin which comprises the steps of (1) and (2).

A human prothrombin gene may be prepared from a whole RNA extracted from a human liver, mRNA or a genomic DNA as a source material by a gene recombination technique well known in the art such as taught by Sambrook et al. (Molecular Cloning, A Laboratory Manual Second Edition. Cold Spring Harbor Laboratory Press, N.Y., 1989). Today, various kits have become commercially available and may be used. For instance, reagents such as TRIzol reagent (Invitrogen), ISOGEN (NIPPON GENE CO., LTD.), and StrataPrep Total RNA Purification Kit (TOYOBO) for extraction of RNAs; kits such as mRNA Purification Kit (Amersham Bioscience), Poly(A) Quick mRNA Isolation Kit (TOYOBO), and mRNA Separator Kit (Clontech) for purification of mRNA; T-Primed First Strand Kit (Amersham Pharmacia), SuperScript plasmid system for cDNA synthesis and plasmid cloning (Invitrogen), cDNA Synthesis Kit (TAKARA SHUZO CO., LTD.), SMART PCR cDNA Synthesis & Library Construction Kits (Clontech), Directionary cDNA Library Construction systems (Novagen Inc.), and GeneAmp PCR Gold (Applied Biosystems) for conversion into cDNA may be used. More specifically, cDNAs are synthesized with mRNAs (Sawady Technology) derived from a human liver as a template using T-Primed First Strand Kit (Amersham Pharmacia), and PCR is performed for amplification of a prothrombin gene using primers PT1 (SEQ ID NO: 1) and PT2 (SEQ ID NO: 2) designed based on the sequence of the prothrombin gene. PT1 and PT2 are added with a Sal restriction enzyme recognition site.

An expression vector for an animal host cell, with no specific limitation, may be defined as a nucleic acid fragment comprising a foreign gene with addition of an expression control region such as a suitable promoter, a termination codon, a polyA addition signal sequence, a Kozak sequence, a secretion signal, and the like. A promoter contained in said expression vector may be any insofar as it leads to expression of a foreign gene, such as an SV40 early promoter, an SV40 late promoter, a Cytomegalovirus promoter, a chicken β-actin promoter, as selected based on an animal cell to be used as a host. Preferably, a chicken β-actin promoter-based expression plasmid pCAGG (Japanese patent publication No. 3-168087) may be used. A marker gene for selection or gene amplification may be one commonly known in the art such as a neo gene, a dihydrofolate reductase (dhfr) gene, a puromycin resistant enzyme gene, or a glutamine synthetase (GS) gene. A commercially available expression vector may be used and includes, for instance, pSI, pCI-neo (Promega) for an animal cell; pPICZ (Invitrogen), pESP-1 (Stratagene) for yeast; BacPAK6 (Clontech), pBAC (Novagen Inc.) for insect cell; pET (Stratagene) for bacteria, which may appropriately be used for the purpose. A modified pCAGG vector was used herein wherein a chicken β-actin promoter-based expression plasmid pCAGG was added with a SalI restriction enzyme recognition site and a neo gene and a dihydrofolate reductase (dhfr) gene as a selection gene.

A host as used herein may be any culture cell from various mammals and includes cells in which a sugar chain structure of N-glycolylneuraminic acid is not added but instead a sugar chain structure of N-acetylneuraminic acid is added, such as Chinese hamster ovary cell (CHO cell), 293 cell derived from human or cells derived from chicken. Gene introduction into an animal cell may be performed, with no specific limitation, by e.g. a phosphate calcium method, a DEAE dextran method, a method using lipofectin liposome, a protoplast polyethylene glycol fusion, electroporation etc., which may suitably be selected depending on a host cell as used (Molecular Cloning (3rd Ed.), Vol. 3, Cold Spring Harbor Laboratory Press (2001)). A medium to be used in culture includes an agar medium, a liquid medium, as classified from its form, or YMM-01, DMEM, RPMI, αMEM, etc. and may suitably be selected depending on a cell, the purpose of culture or a stage of culture. In accordance with respective protocols, a culture medium may be used in which sera, amino acids, vitamins, sugars, antibiotics, pH adjusting buffers and the like are added. A pH of media may be adjusted in a range of 6-8 and a culture temperature may be in a range of 30°C to 39°C. An amount of a medium, any additive and a culture period may suitably be adjusted depending on a culture scale. As an embodiment, a pCAGG modified vector of the invention is introduced into CHO cells by a phosphate calcium method and the cells are continuously cultured in a medium containing Geneticin (GIBCO-BRL) and methotrexate (Wako Pure Chemical Industries, Ltd.) to give a drug-resistant cell. The drug-resistant cell may be cloned by e.g. a limiting dilution.

Cells producing a human prothrombin may be obtained by detecting a human prothrombin present in a culture solution of the cloned drug-resistant cells by a detection method utilizing a specific reaction with an anti-thrombin antibody such as dot blot, Western blot, sandwich ELISA, etc. The thus obtained cells producing a human prothrombin may be adapted to a serum-free culture medium and then subject to culture in a large amount at a level of industrial production. Culture in a large amount may be done by e.g. fed batch culture, batch culture, etc. with no specific limitation.

For purification of a human prothrombin from the cells producing a human prothrombin, a purification method as generally used in protein chemistry may be used such as e.g. a salting out, a ultrafiltration, an isoelectric precipitation, an electrophoresis, an ion exchange chromatography, a gel filtration chromatography, an affinity chromatography, an hydrophobic chromatography, a hydroxyapatite chromatography, etc. In practice, a combination of the above methods may sometimes be employed due to presence of a variety of cellular debris.

An anion exchanger is preferably used in the present invention. An anion exchanger includes a diethylaminoethyl (DEAE) type, a quaternary aminoethyl (QAE) type, etc. A DEAE anion exchanger includes DEAE-agarose (DEAE-Sepharose, Amersham), DEAE-dextran (DEAE-Sephadex, Amersham), DEAE-polyvinyl (DEAE-Toyopearl, Tosoh Corporation), and the like. A QAE anion exchanger includes QAE-agarose (QAE-Sepharose, Amersham), QAE-polyvinyl (QAE-Toyopearl, Tosoh Corporation), and the like. A support as used herein is not specifically limited but QAE-Sepharose (Amersham) is preferably used. A buffer for use in said anion exchanger is not specifically limited and may be any which has a buffering capacity at around a neutral range. A pH range may be 6-8 and a concentration of a buffer may be 0.10 M or less. For instance, phosphoric acid and its salt, citric acid and its salt, maleic acid and its salt, pyrophosphoric acid and its salt, carbonic acid and its salt, glycine, trishydroxymethylaminomethane, as well as any combination of these acids and their salts may be used as appropriate. A salt to be added to a buffer includes sodium chloride, calcium chloride, ammonium sulfate, sodium phosphate, etc. either alone or in combination thereof. More specifically, a human prothrombin may be contacted with a column in a range of pH 7-8, preferably at pH 7.4. A salt concentration may be in a range of from 0.05 M to 0.2 M, preferably 0.1 M. A salt concentration used when a human prothrombin is eluted from an anion exchanger may be in a range of from 0.3 M to 0.5 M, preferably 0.35 M. The thus obtained human prothrombin is highly pure free from contaminants.

Alternatively, a human prothrombin may be purified easily and effectively by Pseudoaffinity in accordance with B. Fischer et al. (Japanese patent publication No. 7-64823). A calcium-binding protein with Gla sequence, upon binding with a bivalent cation such as calcium, alters its properties e.g. steric structure, surface discharge, hydrophilicity etc., affinity with an antibody, and behavior in chromatography e.g. ion exchange, hydrophobic, etc. By taking advantage of this alteration, Pseudoaffinity is a method for specifically purifying a calcium-binding protein with Gla sequence through addition or removal of calcium.

As an example of ion exchange, when prothrombin is subjected to a chromatography using Q-Sepharose-FF (Amersham) as an anion exchanger under conditions of 20 mM Tris-HCl, pH 7.4, it is eluted from a column at NaCl concentration of 0.3 M or more but, upon addition of 10 mM calcium, a manner of prothrombin elution is altered, i.e. it is eluted at a lower NaCl concentration of 0.15 M. Taking advantage of this nature, prothrombin may be adsorbed to an anion exchanger at more than 0.15 M of a salt concentration and, after washing, eluted with a solvent of 0.15 M NaCl containing 10 mM calcium to thereby allow for isolation and removal of impurities having the same isoelectric point as prothrombin and of impurities not isolatable by normal ion exchange chromatography to enable simple preparation of a highly pure prothrombin.

A recombinant ecarin may preferably be used for conversion of a human prothrombin into a human thrombin. A recombinant ecarin may be prepared as described in the patent publication (WO2003/004641). Briefly, a snake venom ecarin cDNA is prepared as taught by Nishida et al. (S. Nishida et al., Biochemistry, 34, 1771-1778, 1995) and incorporated into a chicken β-actin promoter-based expression plasmid pCAGG as used herein. The obtained expression vector is introduced into animal cells, e.g. CHO cells, to provide cells producing a recombinant ecarin. A recombinant ecarin may be purified by a cation exchange chromatography and gel filtration.

The thus purified recombinant ecarin may be added to a solution of the purified human prothrombin for the time sufficient for a conversion reaction. A reaction temperature may be 35-38°C, preferably 37°C, and a reaction time may be 1-4 hours, preferably 2 hours. A human thrombin converted by treatment with ecarin may be purified by a chromatography using benzamidine and a cation exchange column. The thus purified recombinant ecarin may be acted to a human prothrombin for its conversion into a human thrombin. The reaction condition may be the same as in a usual enzymatic reaction. For instance, ecarin at a final concentration of 2-8 Units/mL may be added to a human prothrombin at 1000 µg/mL for reaction at 36-38°C for 1-4 hours to complete conversion of a human prothrombin into a human thrombin.

A human thrombin may be purified by subjecting the solution after treatment with ecarin to the method for purifying a protein as described above. Preferably, a method for purification using a combination of a benzamidine chromatography and a cation exchange chromatography may be used. Like in anion exchange, a buffer may be used which has a buffering capacity at around a neutral range. A pH may be in a range of 7-9, preferably 8, and a concentration of a buffer may be 0.1 M or less, preferably 0.01 M. For adsorption of a human thrombin to a column, a salt of 0.3-0.7 M may be included. Preferably, 0.5 M NaCl may be used. A human thrombin, after washing with the buffer, may be eluted with the buffer as used for adsorption and washing to which 0.1 M benzamidine hydrochloride is added.

For obtaining a human thrombin highly purified, a human thrombin is further subjected to a cation exchange chromatography. A cation exchange chromatography includes a sulfopropyl type, carboxymethyl type, and the like and may be selected as appropriate. SP Toyopearl 550C (Tosoh Corporation) is used in the present invention. The conditions for adsorption, washing and elution may generally be the same as those of a cation exchange chromatography. For example, pH of 6-8, preferably 6-7, and a buffer concentration of 5-200, preferably 10 mM may be used. More specifically, 10 mM citric acid buffer to which 0.2 M NaCl is added may be used. For elution of a human thrombin, a salt concentration may be increased. Preferably, 0.6 M NaCl may be used. By these procedures, a human thrombin of high purity may be obtained.

The thus obtained human thrombin may then be analyzed for its enzymatic chemical property such as clotting activity and the activity to cleave a synthetic substrate (S-2238) and for its sugar chain. A value for the clotting activity represents a time required for clotting by fibrinogen relative to a calibration curve of standard, including thrombin of the Japanese Pharmacopoeia standard and WHO international standard (NIBSC). The activity to cleave S-2238, which is based on a reaction between thrombin and its specific substrate, may be measured by determining an amount of p-nitroaniline released upon cleavage of the synthetic substrate S-2238 by thrombin through a change in absorbance at OD405/650.

Measurement of sialic acid may be performed by using N-Acetylneuraminic Acid and N-Glycolylneuraminic Acid as a standard substance and comparing an elution time of a test substance from an anion exchange chromatography column with that of a standard substance. Thus, a respective regression curve may be obtained from peak areas of standard substances at each concentration. The measurement of a test substance may then be extrapolated into the obtained regression curve to give a concentration of sialic acid.

The present invention is explained in more detail by means of the following Examples which are not intended to restrict a scope of the present invention in any sense. Reagents used in the following Preparation and Examples were obtained from Amersham, BioRad, Wako Pure Chemical Industries, Ltd., TAKARA SHUZO CO., Ltd., Toyobo, and New England BioLabs, unless otherwise instructed.

### Example 1

### (Construction of expression plasmid)

(1) Construction of expression plasmid pCAGG-S1(Sal)
   A chicken β-actin promoter-based expression plasmid pCAGG (Japanese Patent Publication No. 168087/1991) was digested with restriction enzyme EcoRI, blunt-ended with T4 DNA polymerase, and then ligated with T4 DNA ligase in the presence of phosphorylated XhoI linker to construct pCAGG(Xho). The obtained pCAGG(Xho) was digested with restriction enzyme SalI, blunt-ended with T4 DNA polymerase, and then ligated with T4 DNA ligase to construct pCAGG-Pv2. The resulting pCAGG-Pv2 was digested with restriction enzyme XhoI and then treated with S1 nuclease to erase several nucleotides in the vicinity of the XhoI recognition site. After the nuclease treatment, a single chain region was modified with T4 DNA polymerase in the presence of dNTPs and then ligated with T4 DNA ligase in the presence of phosphorylated SalI linker to construct pCAGG-S1(Sal).
(2) Construction of expression plasmid pCAGG-S1(Sal).dhfr
   Expression plasmid pSV2-dhfr bearing DHFR gene (S. Subramani et al., Mol. Cell. Biol., 1, p.854-864, 1981) was digested with restriction enzyme BglII, blunt-ended with T4 DNA polymerase, and ligated with T4 DNA ligase to construct pSV2-dhfr-Bgn. The resulting pSV2-dhfr-Bgn was then digested with restriction enzyme PvuII and ligated with T4 DNA ligase in the presence of phosphorylated BglII linker to construct pSV2-dhfr-BgB. The obtained pSV2-dhfr-BgB was digested with restriction enzymes BglII and BamHI and was then subject to agarose gel electrophoresis to obtain a fragment of about 1.7 kbp. The expression plasmid pCAGG-S1(Sal) obtained above was digested with restriction enzyme BamHI and then ligated to cyclize with the 1.7 kbp fragment to construct pCAGGS1(Sal) .dhfr.
(3) Construction of expression plasmid pCAGG-S1(Sal) .dhfr.neo
   An aminoglycoside phosphotransferase (neo)-based expression plasmid pMClneo-polyA (K. R. Thomas et al., Cell, 51, p.503-512, 1987) was digested with restriction enzyme XhoI and then ligated with T4 DNA ligase in the presence of a phosphorylated BamHI linker to construct pMClneo-2B. The resulting pMClneo-2B was digested with restriction enzyme BamHI and then subject to agarose gel electrophoresis to obtain a fragment of about 1.1 kbp. The expression plasmid pCAGG-S1(Sal).dhfr obtained above was digested with restriction enzyme BamHI and then ligated to cyclize with the fragment of about 1.1 kbp to construct pCAGG-S1(Sal).dhfr.neo.

### Example 2

### (Preparation of human prothrombin gene)

Using human liver mRNAs (Sawady Technology) as a template, 1st strand cDNAs were synthesized by the method known in the art with Oligo dT as a primer using a reverse transcriptase (T-Primed First Strand Kit; Amersham Pharmacia). Based on the cDNAs, primers as described below were designed and used for PCR. A primer of the sequence:
5'-ACGCGTCGACGTCGCCGCCACCATGGCGCACGTCCGAGGCTTGCAGCTGCCTGGCT GC (PT1; SEQ ID NO: 1)
for the gene corresponding to the N-terminal of prothrombin and a primer of the sequence:
5'-GCCGACGTCGACGCGTCTACTCTCCAAACTGATCAATGACCTTCTG (PT2; SEQ ID NO: 2)
for the gene corresponding to the C-terminal of prothrombin were used. PCR was performed with Pyrobest DNA polymerase in accordance with the manufacturer's instruction of this enzyme for 30 cycles for gene amplification.

### Example 3

### (Construction of human prothrombin expression plasmid)

The prothrombin structural gene obtained in Example 2 was incorporated into the plasmid pCAGG-S1(Sal).dhfr.neo as described in Example 1. The plasmid pCAGG-S1(Sal).dhfr.neo was digested with restriction enzyme SalI and then dephosphorylated with bovine small intestinederived alkaline phosphatase. The dephosphorylated plasmid and a fragment from digestion of the prothrombin structural gene obtained in Example 2 with restriction enzyme SalI were ligated to cyclize with T4 DNA ligase to construct pCAGG-S1.dhfr.neo.A-11. A nucleotide sequence of the prothrombin structural gene including the restriction enzyme SalI site of this plasmid was determined by the method known in the art (SEQ ID NO: 3).

### Example 4

### (Expression of human prothrombin using animal cells)

The prothrombin expression plasmid as described in Example 3 was used to transfect CHO K1 (G. Urlaub et al., Somatic cell. Mol. Genet., 12, p.555-566 1986; hereinafter referred to as "CHO") cells by calcium phosphate method (C. Chen et al., Mol. Cell. Biol., 7, 2745-2752, p.1987). The expression plasmid for use in transfection was previously linearized by digestion with restriction enzyme PvuI. Quantification of prothrombin was carried out by sandwich ELISA using an anti-human thrombin antibody.
(1) Performance of production for prothrombin with CHO cells
   Using CHO cells, transfectants were selected from transfection as described below.

On the day previous to transfection, the cells were plated in YMM-01 (nucleic acid free MEM alpha medium with enriched amino acids/vitamins containing insulin, transferrin, ethanolamine and sodium selenite) supplemented with 10% fetal calf serum (FCS; GIBCO-BRL) in 10 cm dish at a cellular density of 5 x 10⁴ cells/dish. After culture at 37°C overnight, the cells were transfected with 20 µg of the linearized expression plasmid pCAGG-S1.dhfr.neo.A-11. After culture in 3% CO₂ incubator at 35°C overnight, the cells were washed with Dulbecco PBS(-) and the culture medium was replaced with YMM-01 medium containing 10% dialyzed FCS and 500 µg/mL Geneticin (GIBCO-BRL), supplement, and 200 nmol/L methotrexate (MTX; Wako Pure Chemical Industries, Ltd.) and culture was continued in 5% CO₂ incubator at 37°C. The emerged transfectants were pooled, expanded and cloned. Each 200 µL/well of the cells were inoculated onto 96 well plate at a concentration of 2.5 cells/well with the same culture medium for limiting dilution. Each of the obtained clones was assayed for an ability to produce prothrombin. Each clone was plated in YMM-01 medium containing 10% dialyzed FCS and supplement at a density of 2 x 10⁵ cells/mL and cultured overnight and the culture medium was replaced with YMM-01 medium free from the dialyzed FCS to adapt the cells to a serum free medium. The cells were then inoculated to a 250 mL shaker flask for batch culture for 14 days in total. A prothrombin level expressed in culture supernatant was measured to reveal that clone A-36 expressed 128 µg/mL of prothrombin in the culture supernatant.

### Example 5

### (Large scale culture of prothrombin-producing cells)

The prothrombin-producing cells A-36 as described in Example 4 were adapted to a serum free medium and then inoculated to a 600 1 fermenter for culture in a self-manufactured serum free medium for 10 days in total. A prothrombin level expressed in culture supernatant was measured to detect expression of 110 µg/mL of prothrombin.

### Example 6

### (Purification of recombinant human thrombin)

(1) Anion exchange chromatography
   To 3200 ml of culture supernatant of the prothrombin-producing CHO cells was added a 2-fold amount of 20 mM phosphate buffer (pH 7.4). The mixture was filtered with a 0.22 µm filter and the filtrate was used as a sample. The sample was applied to a 80 ml column of Q-Sepharose Fast Flow (Amersham) equilibrated with 10 mM phosphate buffer (pH 7.4) containing 0.1M NaCl at a flow rate of 6.6 ml/min. After washing the column with 1600 ml of the buffer, the column was eluted with 450 ml of 10 mM phosphate buffer (pH 7.4) containing 0.35M NaCl at a flow rate of 6.6 ml/min.
(2) Activation of thrombin
   To 360 ml of the eluate of anion exchange chromatography was added a purified recombinant ecarin at a final concentration of 4 U/ml for reaction at 37°C for 2 hours.
(3) Purification with benzamidine column
   The reaction solution after the ecarin treatment obtained in (2) above was applied to a 40 ml column of Benzamidine Fast Flow (Amersham) equilibrated with 10 mM Tris-HCl (pH 8.0) buffer containing 0.5M NaCl at a flow rate of 2.5 ml/min. After washing the column with 800 ml of the buffer, the column was eluted with 360 ml of 10 mM Tris-HCl + 0.5 M NaCl (pH 8.0) buffer containing 100 mM benzamidine hydrochloride.
(4) Cation exchange chromatography
   To 160 mL of the benzamidine column eluate obtained in (3) above was added a 2.5-fold amount of 10 mM citrate buffer (pH7.0) and the mixture was used as a sample. The sample was applied to a 40 ml column of SP Toyopearl 550C (Tosoh Corporation) equilibrated with 10 mM citrate buffer (pH 7.0) containing 0.2M NaCl at a flow rate of 6.6 ml/min. After washing the column with 800 ml of the buffer, the column was eluted with 120 ml of 10 mM citrate buffer (pH 7.0) containing 0.6M NaCl at a flow rate of 6.6 ml/min. As a consequence of the processes described above, a highly purified recombinant human thrombin was obtained at as high a final purification yield as 70%.

### Example 7

### (Analysis of recombinant human thrombin)

The purified recombinant human thrombin obtained as described in Example 6 was analyzed for its enzymatic chemical property and its sugar chain by measuring a clotting activity, an activity to cleave a synthetic substrate S-2238 and sialic acid. A purified human thrombin derived from plasma (purchased from HTI) was used as a positive control. As a result, the purified recombinant human thrombin exhibited equivalent values to the purified human thrombin derived from plasma as shown in Table 1.
(1) Clotting activity
Thrombin to be tested was diluted with a saline containing 1.0% BSA, 100 µL of which was added to a test tube made of polypropylene and heated at 37°C for more than 2 minutes. To the thrombin solution was added 900 µL of 0.1% fibrinogen solution (Juridical Foundation The Chemo-Sero-Therapeutic Research Institute) previously heated at 37°C while avoiding bubbling. After lightly stirring, the mixture was set in a well of Toxinometer (Wako Pure Chemical Industries, Ltd.; ET-201) to measure a time for clotting. Using thrombin of the Japanese Pharmacopoeia' standard and WHO international standard (NIBSC), a calibration curve was prepared and then the measurement of the sample was extrapolated thereto to obtain the clotting activity. The results are shown in Table 1.
(2) Activity to cleave synthetic substrate S-2238
To a 2008 tube manufactured by Falcon were added 20 µl of the sample, 60 µl of 50 mM Tris-HCl (pH 8.5) + 50 mM NaCl buffer and 20 µl of 0.1% PLURONIC F-68 and the mixture was incubated at 37°C for 3 minutes. To the reaction solution was added 100 µl of S-2238 (1 mM: Daiichi Pure Chemicals Co., Ltd.), a TESTZYM chromogenic substrate, and the mixture was stirred for reaction at 37°C for 5 minutes. Then, 800 µl of 0.1 M citrate solution was added to stop the reaction. The reaction solution (200 µl) was transferred to a 96-well plate and OD405/650 was measured. The results are shown in Table 1.

**Table 1**

| | Recombinant thrombin | Plasma-derived thrombin (HTI) |
|---|---|---|
| Clotting activity | | |
| Japan Pharmacopoeia | 1784 U/mg | 1790 U/mg |
| NIBSC | 3218 U/mg | 2937 U/mg |
| Activity to cleave synthetic substrate S-2238 (405/650 value/mg) | 214 | 200 |

(3) Analysis for sialic acid
Ten µL of the thrombin sample, 10 µL of 150 mM sodium citrate and 10 µL of 1 U/mL Neuraminidase were mixed together. The mixture was reacted at 37°C for 3 hours, diluted with purified water and filtered with a 0.45 µm filter and the filtrate was used for analysis. An analytic devise used was DIONEX ION CHROMATOGRAPH. CarboPac PA1 (4 x 250 mm) (DIONEX) was used as a column for separation. Using N-Acetylneuraminic Acid and N-Glycolylneuraminic Acid as a standard substance, their elution time was compared with that of the sample to identify sialic acid in the sample. Respective regression curves were prepared from peak areas of standard substances at each concentration. The measurement of the sample was then extrapolated into the obtained regression curves to calculate a concentration of sialic acid. The results are shown in Table 2.

**Table 2**

| | N-Acetylneuraminic Acid | N-Glycolylneuraminic Acid |
|---|---|---|
| Human thrombin from SP2/0 | 10% | 90% |
| Human thrombin from CHO | 95% | 5% |

### INDUSTRIAL APPLICABILITY

According to the present invention, a human thrombin may be provided at an industrial scale at a low cost that is safe by excluding risk factors such as viruses and prion from blood and has a high biocompatibility to human with respect to its sugar chain.

SEQUENCE LISTING

<110> Juridical Foundation the Chemo-Sero-Therapeutic Research Institute
<120> Process for preparing recombinant human thrombin by culture cells
<130> 666958
<150> JP 2005-289076
<151> 2005-09-30
<160> 3
<170> PatentIn version 3.1

<210> 1
<211> 58
<212> DNA
<213> Artificial

<220>
<223> Sense primer for amplifying a prothrombin structural gene from human
liver-drived cDNA by PCR

<400> 1
acgcgtcgac gtcgccgcca ccatggcgca cgtccgaggc ttgcagctgc ctggctgc       58


<210> 2
<211> 46
<212> DNA
<213> Artificial

<220>
<223> Antisense primer for amplifying a prothrombin structural gene from human
liver-drived cDNA by PCR

<400> 2
gccgacgtcg acgcgtctac tctccaaact gatcaatgac cttctg                    46


<210> 3
<211> 1898
<212> DNA
<213> Homo sapiens

<400> 3
gtcgacgtca gccgccacca tggctcacgt ccgaggcttg cagctgcctg gctgcctggc     60
cctggctgcc ctgtgtagcc ttgtgcacag ccagcatgtg ttcctggctc ctcagcaagc    120
acggtcgctg ctccagcggg tccggcgagc caacaccttc ttggaggagg tgcgcaaggg    180
caacctggag cgagagtgcg tggaggagac gtgcagctac gaggaggcct tcgaggctct    240
ggagtcctcc acggctacgg atgtgttctg ggccaagtac acagcttgtg agacagcgag    300
gacgcctcga gataagcttg ctgcatgtct ggaaggtaac tgtgctgagg gtctgggtac    360
gaactaccga gggcatgtga acatcacccg gtcaggcatt gagtgccagc tatggaggag    420
tcgctaccca cataagcctg aaatcaactc cactacccat cctggggccg acctacagga    480
gaatttctgc cgcaaccccg acagcagcac cacgggaccc tggtgctaca ctacagaccc    540
caccgtgagg aggcaggaat gcagcatccc tgtctgtggc caggatcaag tcactgtagc    600
gatgactcca cgctccgaag gctccagtgt gaatctgtca cctccattgg agcagtgtgt    660
ccctgatcgg gggcagcagt accaggggcg cctggcggtg accacacatg ggctcccctg    720
cctggcctgg gccagcgcac aggccaaggc cctgagcaag caccaggact tcaactcagc    780
tgtgcagctg gtggagaact tctgccgcaa cccagacggg gatgaggagg gcgtgtggtg    840
ctatgtggcc gggaagcctg gcgactttgg gtactgcgac ctcaactatt gtgaggaggc    900
cgtggaggag gagacaggag atgggctgga tgaggactca gacagggcca tcgaagggcg    960
taccgccacc agtgagtacc agactttctt caatccgagg acctttggct cgggagaggc   1020
agactgtggg ctgcgacctc tgttcgagaa gaagtcgctg gaggacaaaa ccgaaagaga   1080
gctcctggaa tcctacatcg acgggcgcat tgtggagggc tcggatgcag agatcggcat   1140
gtcaccttgg caggtgatgc ttttccggaa gagtccccag gagctgctgt gtggggccag   1200
cctcatcagt gaccgctggg tcctcaccgc cgcccactgc ctcctgtacc cgccctggga   1260
caagaacttc accgagaatg accttctggt gcgcattggc aagcactccc gcaccaggta   1320
cgagcgaaac attgaaaaga tatccatgtt ggaaaagatc tacatccacc ccaggtacaa   1380
ctggcgggag aacctggacc gggacattgc cctgatgaag ctgaagaagc ctgttgcctt   1440
cagtgactac attcaccctg tgtgtctgcc cgacagggag acggcagcca gcttgctcca   1500
ggctggatac aagggggcggg tgacaggctg gggcaacctg aaggagacgt ggacagccaa   1560

666958.txt
cgttggtaag gggcagccca gtgtcctgca ggtggtgaac ttgcccattg tggagcggcc 1620
ggtctgcaag gactccaccc ggatccgcat cactgacaac atgttctgtg ctggttacaa 1680
gcctgatgaa gggaaacgag gggatgcctg tgaaggtgac agtgggggac cctttgtcat 1740
gaagagcccc tttaacaacc gctggtatca aatgggcatc gtctcatggg gtgaaggctg 1800
tgaccgggat gggaaatatg gcttctacac acatgtgttc cgcctgaaga agtggataca 1860
gaaggtcatt gatcagtttg gagagtagac gcgtcgac 1898

## Claims

1. A process for preparing a recombinant human thrombin which comprises a series of the following steps (1) to (4) :
(1) a step of obtaining a transfectant cell producing a human prothrombin by introducing an expression vector, wherein a gene fragment coding for a human prothrombin gene is incorporated, into an animal cell;
(2) a step of purifying a human prothrombin from the culture of the transfectant cell above by an anion exchanger;
(3) a step of converting the purified human prothrombin into a human thrombin by subjecting said human prothrombin to the action of ecarin; and
(4) a step of purifying the human thrombin from the solution after treatment with ecarin by an affinity method using benzamidine and a cation exchanger.

2. The process according to claim 1 wherein the animal cell is a CHO cell.

3. The process according to claim 1 or 2 wherein the human prothrombin gene consists of the nucleotide sequence as depicted in SEQ ID NO: 3.

4. A process for preparing a recombinant human prothrombin which comprises a series of the following steps (1) to (2):
(1) a step of obtaining a transfectant cell producing a human prothrombin by introducing an expression vector, wherein a gene fragment coding for a human prothrombin gene is incorporated, into an animal cell; and
(2) a step of purifying a human prothrombin from the culture of the transfectant cell above by an anion exchanger.

5. The process according to claim 4 wherein the animal cell is a CHO cell.

6. A human thrombin obtained by the process as set forth in any of claims 1 to 3.

7. A human prothrombin obtained by the process as set forth in claim 4 or 5.

8. A CHO cell that produces a human prothrombin.
